## Europäisches Patentamt
## European Patent Office
## Office européen des brevets

(19)

(11) Veröffentlichungsnummer: **0 047 830**
**B1**

(12) **EUROPÄISCHE PATENTSCHRIFT**

(45) Veröffentlichungstag der Patentschrift:
02.03.83

(51) Int. Cl.³: **C 07 C 67/38,** B 01 J 31/28,
B 01 J 31/40

(21) Anmeldenummer: 81105438.6

(22) Anmeldetag: 11.07.81

(54) **Verfahren zur Herstellung von Alkylestern gesättigter aliphatischer Carbonsäuren.**

(30) Priorität: 12.09.80 DE 3034420

(43) Veröffentlichungstag der Anmeldung:
24.03.82 Patentblatt 82/12

(45) Bekanntmachung des Hinweises auf die Patenterteilung:
02.03.83 Patentblatt 83/9

(84) Benannte Vertragsstaaten:
AT BE FR GB IT NL

(56) Entgegenhaltungen:
EP-A-0 008 024
EP-A-0 021 010
DE-A-2 503 996
DE-A-2 912 489
Patents Abstracts of Japan Band 5, Nr. 12, 24.
Januar 1981

(73) Patentinhaber: CHEMISCHE WERKE HÜLS AG,
Postfach 1320, D-4370 Marl 1 (DE)

(72) Erfinder: Hofmann, Peter, Dr., Lipper Weg 193,
D-4370 Marl 1 (DE)
Erfinder: Müller, Wolfgang Hans Eduard, Dr., Bitterfelder
Strasse 9a, D-4370 Marl 1 (DE)

## Verfahren zur Herstellung von Alkylestern gesättigter aliphatischer Carbonsäuren

Es ist bekannt, daß man durch Umsetzung von Olefinen mit Kohlenmonoxid und H-aciden Komponenten, wie z. B. Alkanolen, in Gegenwart eines Katalysators, der ein Metall der 8. Nebengruppe des Periodischen Systems der Elemente und gegebenenfalls einen Promotor enthält, Fettsäureester herstellen kann (J. Falbe, Synthesen mit Kohlenmonoxid, Springer-Verlag, Berlin, Heidelberg, New York, 1967).

Eine bevorzugte Variante dieser als Hydrocarboxilierung bezeichneten Reaktion ist die Umsetzung in Gegenwart von kobalthaltigen Katalysatoren. Als besonders bevorzugte Ausführungsform hat sich der zusätzliche Einsatz von Pyridin oder einem nicht-ortho-substituierten Alkylpyridin als Promotor erwiesen (US-PS 3 507 891).

Ein wesentliches Problem dieser homogen katalysierten Reaktion ist die Rückgewinnung des relativ teuren Pyridins und/oder nicht-ortho-substituierten Alkylpyridins aus dem Reaktionsgemisch in einer Form, die seinen (ihren) Wiedereinsatz als Promotor gestattet.

Aus der DE-OS 1 963 804 und der US-PS 3 507 891 ist es lediglich bekannt, daß die Abtrennung des Promotors durch Destillation erfolgen kann.

Bei mehrmaliger Wiederverwendung eines so zurückgewonnenen Promotors kommt es jedoch — wie eigene Versuche gezeigt haben — im Laufe der Zeit zu einem Umsatzrückgang, der durch eine Desaktivierung des Katalysatorsystems, vornehmlich durch eine Anreicherung inhibierend wirkender Substanzen im rückgeführten Promotor bedingt sein dürfte. Es gelang jedoch nicht, diese offenbar in geringer Menge vorhandenen Inhibitoren mit einem wirtschaftlich zu vertretenden Aufwand destillativ zu entfernen.

Aufgabe der vorliegenden Erfindung war es daher, ein Verfahren zu entwickeln, das es gestattet, das als Katalysatorbestandteil (Promotor) eingesetzte Pyridin und/oder nicht-ortho-substituierte Alkylpyridin in einfacher und wirtschaftlicher Weise so zu reinigen, daß es ohne Beeinträchtigung der Aktivität des Katalysatorsystems in den Hydrocarboxilierungsprozeß zurückgeführt werden kann.

Diese Aufgabe wurde durch die in den Patentansprüchen beschriebenen Maßnahmen gelöst.

Das erfindungsgemäße Verfahren kann im Prinzip bei allen Hydrocarboxilierungsverfahren zur Herstellung von Fettsäureestern angewandt werden, bei denen ein Katalysator, bestehend aus einer Kobaltverbindung und Pyridin und/oder einem nicht-ortho-substituierten Alkylpyridin, eingesetzt wird (z. B. Verfahren gemäß US-PS 3 507 891 und deutscher Patentanmeldung P 2 912 489.8). So ist vor allem die Wahl des eingesetzten Olefins unkritisch, d. h. es können sowohl geradkettige oder verzweigte $\alpha$-Olefine als auch Olefine mit innenständiger Doppelbindung eingesetzt werden. Aber auch Olefine mit mehr als einer Doppelbindung und solche mit bestimmten Substituenten, wie z. B. Arylgruppen, sind geeignet.

Im allgemeinen werden Olefine mit 2 bis 40, vorzugsweise mit 4 bis 20, besonders bevorzugt mit 10 bis 18 Kohlenstoffatomen, eingesetzt, die nach bekannten Verfahren des Standes der Technik erhalten werden können. So können z. B. $\alpha$-Olefine durch Oligomerisierung von Ethylen nach Ziegler (DE-PS 878 560 und 1 190 930) oder durch Wachscracken, Olefine mit innenständiger Doppelbindung durch Dehydrierung oder Chlorierung und anschließender Dehydrochlorierung von Paraffinen (GB-PS 1 037 868) gewonnen werden.

Bei dem zuletzt genannten Verfahren werden in der Regel Paraffinschnitte, d. h. Gemische unterschiedlicher C-Zahl eingesetzt, so daß auch die erhaltenen Olefine keine einheitliche C-Zahl aufweisen.

Außerdem kommen in diesen Olefin-Gemischen natürlich alle denkbaren isomeren Formen vor. Neben den reinen — gegebenenfalls substituierten — Olefinen können auch solche mit einem Gehalt an Paraffinen, z. B. bis zu 85 Gewichtsprozent, eingesetzt werden. Der Paraffingehalt rührt daher, daß bei der Olefinherstellung keine vollständigen Umsätze erreicht und die nicht umgesetzten Paraffine nicht oder nicht vollständig abgestrennt werden.

Nicht nur das eingesetzte Olefin, sondern auch die Art des Alkanols, das mit dem Olefin und Kohlenmonoxid umgesetzt wird, ist für das erfindungsgemäße Verfahren unkritisch. Im allgemeinen werden Alkanole mit 1 bis 20, vorzugsweise 1 bis 3 Kohlenstoffatomen, eingesetzt. Typische Vertreter aus der Gruppe der primären Alkanole sind z. B. Methanol, Ethanol und Propanol-(1).

Weiterhin ist es unwesentlich, welche Kobaltverbindung bei der Hydrocarboxilierung verwendet wird. Carbonyle des Kobalts, z. B. Dikobaltoctacarbonyl, sind ebenso geeignet wie carbonsaure Kobaltsalze, wie z. B. Kobaltacetat, Kobaltnaphthenat und Kobalt-2-ethylhexanoat, und Salze des Kobalts mit anorganischen Säuren, wie z. B. Kobaltnitrat und Kobaltsulfat. Vorzugsweise kommen solche carbonsauren Kobaltsalze zum Einsatz, deren Anionen dem Säurerest der bei der Hydrocarboxilierung gebildeten Fettsäureester entsprechen.

Als Promotoren kommen neben Pyridin allein oder im Gemisch alle nicht-ortho-substituierten Alkylpyridine, wie z. B. 3- und 4-Picolin, 3,4- und 3,5-Dimethylpyridin und 3- und 4-Ethylpyridin infrage.

Auch die Reaktionsbedingungen, unter denen die Hydrocarboxilierung durchgeführt wird, sind für das erfindungsgemäße Verfahren nicht von Bedeutung. Im allgemeinen werden Hydrocarboxilierungsverfahren bei Temperaturen von 80 bis 300, vorzugsweise 150 bis 220 C, und Kohlenmonoxiddrucken von 10 bis 800, vorzugsweise 100 bis 300 bar, durchgeführt.

Schließlich ist es nicht verfahrenskritisch, jedoch vorteilhaft, wenn man das Reaktionsgemisch vor der weiteren Aufarbeitung mit Sauerstoff oder einem sauerstoffhaltigen Gas, vorzugsweise Luft, bei Temperaturen von 20 bis 150, vorzugsweise 50 bis 120° C, behandelt. Diese Behandlung, die bereits in der US-PS 3 507 891, Spalte 4, Zeilen 21 bis 43, und in der nicht zum Stand der Technik gehörenden deutschen Patentanmeldung P 2 912 489.8, Seite 5, Absatz 2, beschrieben ist, wird so lange durchgeführt, bis die bei der folgenden destillativen Aufarbeitung zur Abscheidung von metallischem Kobalt führenden Kobaltverbindungen oxidativ zerstört sind.

Bei der sich anschließenden destillativen Aufarbeitung werden nicht umgesetztes Alkanol und Olefin, der Promotor und die Reaktionsprodukte in einem Schritt oder stufenweise bei Sumpftemperaturen bis maximal 350° C abgetrennt. Bevorzugt ist die stufenweise Aufarbeitung, weil man so Fraktionen erhält, die man mit Ausnahme der Fettsäureester an geeigneter Stelle in den Prozeß zurückführen kann.

Überraschend wurde nun gefunden, daß mit Hilfe der erfindungsgemäßen Maßnahme das als Katalysatorbestandteil eingesetzte Pyridin und/oder nicht-ortho-substituierte Alkylpyridin durch Rektifikation so weit gereinigt werden kann, daß auch bei sehr oft wiederholter Rückführung des Promotors in die Hydrocarboxilierungsstufe, d. h. in die eigentliche Estersynthese, keine erkennbare Aktivitätsminderung des Katalysatorsystems beobachtet wird.

Wichtig ist, daß die bei dem erfindungsgemäßen Verfahren zum Einsatz gelangende Carbonsäure bestimmte Bedingungen erfüllt. So muß sie zunächst unter den Bedingungen der Aufarbeitung des Reaktionsgemisches thermisch stabil sein. Weiterhin ist es erforderlich, daß die Carbonsäure mit dem Promotor unter den für die Rückgewinnung des Promotors angewandten Reaktionsbedingungen ein Maximumazeotrop (Siedepunktsmaximum) bildet.

Sofern das erfindungsgemäße Verfahren auf solche Hydrocarboxilierungsprozesse angewendet wird, bei denen Olefine mit 10 bis 18 Kohlenstoffatomen, Alkanole mit 1 bis 3 Kohlenstoffatomen und als Promotoren Pyridin, 3-Picolin, 4-Picolin, 3,4-Dimethylpyridin, 3,5-Dimethylpyridin, 3-Ethylpyridin oder 4-Ethylpyridin eingesetzt werden, ist es vorteilhaft, daß die Carbonsäure bzw. das Azeotrop dieser Carbonsäure mit den anwesenden Kohlenwasserstoffen (Olefine plus gegebenenfalls Paraffine) niedriger als die Kohlenwasserstoffe siedet. Auf diese Weise ist es möglich, die Abtrennung des Promotors von den Kohlenwasserstoffen und die Reinigung des Promotors mit Hilfe der Carbonsäure in einer Rektifikationsstufe vorzunehmen.

Im Prinzip können beim erfindungsgemäßen Verfahren alle Carbonsäuren eingesetzt werden, die die angegebenen Bedingungen erfüllen. Besonders geeignet sind Essigsäure, Propionsäure sowie n- und iso-Buttersäure.

Um eine Verunreinigung des rückgeführten Promotors mit Carbonsäure zu vermeiden, soll diese spätestens bei der Rektifikation des Promotors in einer solchen Menge zugesetzt werden, die geringer ist als sie der azeotropen Zusammensetzung im System Carbonsäure/Pyridin und/oder nicht-ortho-substituiertem Alkylpyridin unter den Bedingungen der Rektifikation entspricht, und die wenigstens 5 Gew.-ppm, bezogen auf die pro Stunde durchgesetzte Menge an Pyridin und/oder nicht-ortho-substituiertem Alkylpyridin, beträgt.

Im allgemeinen wird das erfindungsgemäße Verfahren, das sowohl diskontinuierlich als auch kontinuierlich betrieben werden kann, jedoch vorzugsweise kontinuierlich durchgeführt wird, folgendermaßen praktiziert:

Das die Hydrocarboxilierungsstufe verlassende Reaktionsgemisch wird zunächst zweckmäßigerweise zur Zerstörung flüchtiger Kobaltverbindung mit Sauerstoff oder einem sauerstoffhaltigen Gas, vorzugsweise Luft, behandelt. Sodann wird das Reaktionsgemisch entsprechend seiner Zusammensetzung und der Lage der Siedepunkte der einzelnen Komponenten — vorzugsweise destillativ — soweit wie erforderlich zerlegt. Die nicht umgesetzten Ausgangsstoffe (Olefin, Alkanol, Promotor und Kobaltverbindung) können gegebenenfalls nach Ausschleusung von Teilmengen und Reinigung bzw. Aufarbeitung in die Hydrocarboxilierungsstufe zurückgeführt werden. Was die Abtrennung und die Reinigung des Promotors anbelangt, so können sie je nach Zusammensetzung des Reaktionsgemisches sowohl ein- als auch mehrstufig, z. B. zweistufig, vorgenommen werden. Je nachdem, ob die Abtrennung und Reinigung des Promotors in einer oder mehreren Stufen vorgenommen wird, fallen die zu entfernenden inhibierend wirkenden Substanzen zusammen mit der vorher diskontinuierlich oder kontinuierlich zugeführten Carbonsäure entweder im Mittelteil oder im Sumpf der Rektifikationskolonne an. Hieraus können sie sowohl flüssig als auch gasförmig diskontinuierlich oder kontinuierlich entnommen und anschließend verworfen werden. Weitere Einzelheiten bezüglich der Durchführung des erfindungsgemäßen Verfahrens können z. B. der Monographie »Industrielle Destillation« von Reinhard Billet, Verlag Chemie, Weinheim (1973), entnommen werden.

Das erfindungsgemäße Verfahren wird anhand der nachfolgenden Beispiele näher erläutert.


Beispiel 1


Nachdem der durch Umsetzung eines überwiegend linearen $C_{11}-C_{13}$-Olefinschnittes mit Methanol

und Kohlenmonoxid mit Kobalt und Picolin-4 als Katalysatorbestandteile nach dem sogenannten Hydrocarboxilierungsverfahren erhaltene Reaktoraustrag nach der Zerstörung der flüchtigen Kobaltverbindungen durch Sauerstoffeinwirkung durch Destillation in ein die weitaus überwiegende Menge der flüchtigen Stoffe aus dem Rohprodukt enthaltendes kobaltfreies Destillat und ein das gesamte im Rohprodukt anwesende Kobalt enthaltendes Konzentrat getrennt wurde, wurden zunächst aus dem kobaltfreien Destillat durch kontinuierliche Rektifikation das im Rohprodukt noch enthaltende Methanol sowie geringe Mengen niedriger als Picolin-4 siedende Stoffe abgetrennt.

Das verbleibende Produkt, das hauptsächlich aus Picolin-4, $C_{11}-C_{13}$-Olefinen, $C_{11}-C_{13}$-Paraffinen sowie den gewünschten $C_{12}-C_{14}$-Carbonsäuremethylestern besteht, wurde kontinuierlich in einer Kolonne mit 65 praktischen Böden bei einem Druck am Kolonnenkopf von 40 mbar rektifiziert. Der Zulauf wurde flüssig mit Siedetemperatur auf den 45. Boden von unten geleitet. Am Kolonnenkopf wurde das Picolin-4 mit einem Rücklaufverhältnis von 5 abgenommen. Aus dem Sumpf der Kolonne wurden die Kohlenwasserstoffe, die Carbonsäuremethylester und geringe Anteile höhersiedender Stoffe unbekannter Struktur, die in weiteren Rektifikationen getrennt wurden, entnommen. Die Temperatur am Kolonnenkopf betrug dabei 56°C, während bei einem Differenzdruck der Kolonne von 85 mbar die Sumpftemperatur 148°C zeigte. Das Temperaturprofil in der Rektifikationskolonne ist in der Spalte 1 der Tabelle 1 eingegeben.

Mit dem Zulauf wurden der Kolonne 0,05 Gewichtsprozent Essigsäure, bezogen auf das anwesende Picolin, kontinuierlich zugeführt. Am 40. Boden von unten gerechnet, wurden, bezogen auf die insgesamt zugeführte Picolinmenge, 0,15 Gewichtsprozent eines nach Kondensation zwei flüssige Phasen bildenden Gemisches dampfförmig entnommen, um eine Anreicherung der schädlichen Stoffe und der Essigsäure in der Kolonne zu vermeiden. Bei diesen Versuchsbedingungen blieb über 50 vollständige Rückführungen des Picolins und des Kobalts in die Synthese die Aktivität und Selektivität des Katalysatorsystems unverändert.

## Vergleichsbeispiel

Bei einer Arbeitsweise, die genau der Arbeitsweise des Beispiels 1 entspricht, bei der jedoch der kontinuierlich betriebenen Rektifikationskolonne keine Essigsäure zugeführt wurde und keinerlei Produkt vom 40. Boden der Kolonne entnommen wurde, stellte sich ein stationäres Temperaturprofil entsprechend der Spalte 2 der Tabelle 1 ein. Nach 20 Rückführungen des zurückgewonnenen Picolins in die Synthese wurde ein Abfall der Aktivität des Katalysatorsystems auf 62% der Anfangsaktivität und nach 30 Rückführungen ein Abfall der Aktivität auf 39% der Anfangsaktivität festgestellt.

## Beispiel 2

Bei einer Arbeitsweise entsprechend der Arbeitsweise in Beispiel 1, bei der jedoch keine Essigsäure mit dem Zulauf der kontinuierlich betriebenen Rektifikationskolonne zugeführt wurde, sondern 0,05 Gewichtsprozent Buttersäure, bezogen auf die zugeführte Picolinmenge, in der Kolonne auf den 50. Boden von unten gezählt gegeben wurden und wie in Beispiel 1 vom 40. Boden dampfförmig 0,15 Gewichtsprozent Produkt aus der Kolonne entnommen wurden, stellte sich das in der Tabelle 1, Spalte 3, angegebene Temperaturprofil ein. Die Aktivität und Selektivität des Katalysatorsystems war bei der Hydrocarboxilierungsreaktion auch nach 32 vollständigen Rückführungen des Kobalts und des Picolins unverändert.

Tabelle 1

| | Beispiel Nr. | | |
| --- | --- | --- | --- |
| | 1 | 2 | 3 |
| Gesamtbodenzahl | 65 | 65 | 65 |
| Zulaufboden*) | 45 | 45 | 45 |
| Abnahmeboden*) | 40 | 40 | 40 |
| Buttersäurezuleitung*) | — | — | 50 |
| Druck am Kolonnenkopf [mbar] | 40 | 40 | 40 |
| Druck am Kolonnensumpf [mbar] | 125 | 125 | 125 |
| Rücklaufverhältnis | 5 | 5 | 5 |
| Temperatur Kolonnenkopf | 56 | 56 | 56 |
| Temperatur 60. Boden | 59 | 59 | 59 |
| Temperatur 55. Boden | 70 | 62 | 92 |
| Temperatur 50. Boden | 79 | 65 | 103 |
| Temperatur 45. Boden | 86 | 80 | 111 |
| Temperatur 43. Boden | 92 | 83 | 112 |
| Temperatur 40. Boden | 94 | 89 | 113 |
| Temperatur 30. Boden | 105 | 108 | 115 |
| Temperatur 20. Boden | 117 | 117 | 117 |
| Temperatur 10. Boden | 125 | 126 | 126 |
| Temperatur Sumpf | 148 | 148 | 148 |

*) Kolonnenböden von unten gezählt.

## Patentansprüche

1. Verfahren zur Herstellung von Alkylestern gesättigter aliphatischer Carbonsäuren durch Umsetzung von Olefinen mit Kohlenmonoxid und Alkanol in Gegenwart eines Katalysators, bestehend aus einer Kobaltverbindung und Pyridin und/oder einem nicht-ortho-substituierten Alkylpyridin bei erhöhtem Druck und erhöhter Temperatur, dadurch gekennzeichnet, daß man bei der Aufarbeitung des Reaktionsgemisches das als Katalysatorbestandteil (Promotor) eingesetzte Pyridin und/oder nicht-ortho-substituierte Alkylpyridin vor seiner Rückführung in die Hydrocarboxilierungsstufe in Gegenwart einer solchen unter den Aufarbeitungsbedingungen thermisch stabilen Carbonsäure rektifiziert, die mit dem Pyridin und/oder dem nicht-orthosubstituierten Alkylpyridin unter den Bedingungen der Rektifikation ein Maximumazeotrop bildet.

2. Verfahren nach Anspruch 1, dadurch gekennzeichnet, daß man die Carbonsäure in einer solchen Menge zusetzt, die geringer ist als sie der azeotropen Zusammensetzung im System Carbonsäure/Pyridin und/oder nicht-ortho-substituiertem Alkylpyridin unter den Bedingungen der Rektifikation entspricht, und die wenigstens 5 Gew.-ppm, bezogen auf die pro Stunde durchgesetzte Menge an Pyridin und/oder nicht-ortho-substituiertem Alkylpyridin, beträgt.

3. Verfahren nach den Ansprüchen 1 und 2, dadurch gekennzeichnet, daß man Olefin mit 10 bis 18 Kohlenstoffatomen, Alkanole mit 1 bis 3 Kohlenstoffatomen, als Promotor Pyridin, 3-Picolin, 4-Picolin, 3,4-Dimethylpyridin, 3,5-Dimethylpyridin, 3-Ethylpyridin oder 4-Ethylpyridin und eine solche Carbonsäure einsetzt, die mit dem Promotor ein Maximum- und den anwesenden Kohlenwasserstoffen ein Minimumazeotrop bildet oder niedriger als diese siedet.

4. Verfahren nach den Ansprüchen 1 bis 3, dadurch gekennzeichnet, daß man eine Carbonsäure mit 2 bis 4 Kohlenstoffatomen einsetzt.

5. Verfahren nach Anspruch 4, dadurch gekennzeichnet, daß man als Carbonsäure Essigsäure einsetzt.

## Claims

1. A process for the manufacture of an alkyl ester of a saturated aliphatic carboxylic acid by reaction of an olefin with carbon monoxide and an alkanol in the presence of a catalyst comprising a cobalt compound and pyridine and/or an alkylpyridine other than an ortho-substituted alkylpyridine at elevated pressure and elevated temperature, characterised in that on working up of the reaction mixture the pyridine and/or non-ortho-substituted alkylpyridine used as a catalyst component (promoter) is rectified, prior to its recycle to the hydrocarboxylation stage, in the presence of a carboxylic acid which is thermally stable under the working-up conditions and which forms an azeotrope of maximum boiling point with the pyridine and/or non-orthosubstituted alkylpyridine under the rectification conditions.

2. A process according to claim 1, characterised in that the carboxylic acid is used in an amount which is less than the amount corresponding to the azeotropic concentration in the system carboxylic acid/pyridine and/or non-orthosubstituted alkylpyridine under the rectification conditions but is at least 5 ppm by weight based on the throughput per hour of pyridine and/or non-ortho-substituted alkylpyridine.

3. A process according to claim 1 or 2, characterised by the use of an olefin of 10 to 18 carbon atoms, an alkanol of 1 to 3 carbon atoms, a promoter selected from pyridine, 3-picoline, 4-picoline, 3,4-dimethylpyridine, 3,5-dimethylpyridine, 3-ethylpyridine and 4-ehylpyridine, and a carboxylic acid which forms an azeotrope of maximum boiling point with the promoter and forms an azeotrope of minimum boiling point with the hydrocarbon used or boils lower than it.

4. A process according to any of claims 1 to 3, characterised in that a carboxylic acid of 2 to 4 carbon atoms is used.

5. A process according to claim 4, characterised in that acetic acid is used as the carboxylic acid.

## Revendications

1. Procédé de préparation d'esters alkyliques d'acides carboxyliques aliphatiques saturés, par réaction d'oléfines sur du monoxyde de carbone et un alcanol en présence d'un catalyseur constitué par un composé du cobalt et de la pyridine et/ou une alkyl-pyridine non-substituée en position ortho, sous haute pression et à température élevée, le dit procédé étant caractérisé par le fait que, lors du traitement du mélange réactionnel, la pyridine et/ou l'alkyl-pyridine non-substituée en position ortho urilisée comme donstituant de catalyseur (promoteur), avant d'être renvoyée dans l'étage d'hydrocarboxylation, est rectifiée en présence d'un acide carboxylique qui est thermiquement stable dans les conditions du traitement et qui, dans les conditions de la rectification, forme un azéotrope maximal avec la pyridine et/ou l'alkyl-pyridine non-substituée en position ortho.

2. Procédé selon la revendication 1, caractérisé par le fait qu'on ajoute l'acide carboxylique dans une quantité moindre que celle correspondant, dans les conditions de la réaction, à la composition azéotropique dans le système acide carboxylique/pyridine et/ou alkyl-pyridine non-substituée en position ortho et qui, relativement à la quantité de pyridine et/ou d'alkyl-pyridine non-substituée en position ortho qui passe par heure, est d'au moins 5 ppm en poids.

3. Procédé selon les revendications 1 et 2, caractérisé par le fait qu'on utilise des oléfines comportant de 10 à 18 atomes de carbone, des alcanols comportant de 1 à 3 atomes de carbone, qu'on utilise comme promoteur la pyridine, la 3-picoline, la 4-picoline, la 3,4-diméthyl-pyridine, la 3,5-diméthyl-pyridine, la 3-éthyl-pyridine ou la 4-éthyl-pyridine et qu'on utilise un acide carboxylique qui forme avec le promoteur un azéotrope maximal et avec les hydrocarbures présents un azéotrope minimal ou qui présente un point d'ébullition inférieur à ceux-ci.

4. Procédé selon les revendications 1 à 3, caractérisé par le fait qu'on utilise un acide carboxylique comportant de 2 à 4 atomes de carbone.

5. Procédé selon la revendication 4, caractérisé par le fait qu'on utilise, en tant qu'acide carboxylique, l'acide acétique.